# EUROPEAN PATENT APPLICATION

(11) **EP 1 529 539 A1**
(43) Date of publication of application: **11.05.2005**
(21) Application number: 04256695.0
(22) Date of filing: 29.10.2004
(51) Int. Cl.: A61L 2/26, A61L 2/20, B65D 33/00, B65B 55/18

(54) **Sterilization packaging with friction enhancing material**

(30) Priority: 31.10.2003 US 699271
(71) Applicant: ETHICON, INC., Somerville, NJ 08876 (US)
(72) Inventor: Wi, Su-Syin, Irvine, CA 92614 (US)
(74) Representative: Mercer, Christopher Paul

(57) **Abstract**

A sterilization package encloses a device during a sterilization procedure and stores the device in sterile form thereafter. The package comprises a barrier film which defines an interior space (20) and seals the interior space from contaminating microorganisms. At least a portion of the barrier film is formed of a semi-permeable material (12) which is permeable to sterilizing gasses and impermeable to contaminating microorganisms. At least a portion (Fig.2) of an outer surface of the barrier film has thereon a friction enhancing material.

## Description

### BACKGROUND

This application relates to packaging for goods during a sterilization process, and more specifically to such packaging, which carries a friction enhancement.

Prior to sterilization, medical instruments or other devices which are to be sterilized are typically placed into packages which protect the sterility of the device after sterilization yet allow the sterilization process to effect sterilization of the device.

One popular packaging system employs two film layers, one film formed of a laminate of clear polyolefin and polyester, and another formed of semi-permeable film such as TYVEK™ (spun-bond polyethylene) or medical grade paper. A peripheral seal, such as with adhesive or melt bonding, attaches the two films together and defines an interior space between them which receives the device to be sterilized. The TYVEK™ layer is permeable to sterilizing gases such as vapor phase hydrogen peroxide, ozone, ethylene oxide, and steam, yet impermeable to potentially contaminating microorganisms. Similarly, the medical grade paper layer is permeable to sterilizing gases such as ethylene oxide, ozone and steam, yet impermeable to potentially contaminating microorganisms. Such packages are inexpensive and work quite efficiently to allow sterilization of devices and to protect the sterility of those devices after the sterilization procedure is complete.

These and similar packages are notoriously difficult to stack as the materials from which they are formed have rather low coefficients of friction. The present invention overcomes this limitation without impacting the ability of the packaging to be used in a variety of sterilization processes (i.e. steam, vapor phase hydrogen peroxide and ethylene oxide) and without adversely affecting the ability of sterilant vapor to penetrate the package and effect sterilization of the device.

### SUMMARY OF INVENTION

A sterilization package according to the present invention is for enclosing a device during a sterilization procedure and storing the device in sterile form thereafter. The package comprises a barrier film defining an interior space and sealing the interior space from contaminating microorganisms. At least a portion of the barrier film is formed of a semi-permeable material which is permeable to sterilizing gasses and impermeable to contaminating microorganisms. At least a portion of an outer surface of the package carries thereon a friction enhancing material.

Preferably, the barrier film is flexible. In one preferred embodiment of the sterilization package, the barrier film is formed of two separate film panels sealed together. Preferably, both film panels have the friction enhancing material thereon. Preferably, at least one of the film panels consists of the semi-permeable material a portion of which has the friction enhancing material thereon. The package is primarily intended to contain a medical instrument.

Preferably, the friction enhancing material comprises an elastomer, as for example silicone.

A portion of the semi-permeable material can have the friction enhancing material thereon. Preferably, it is applied in a pattern that leaves a portion of the semi-permeable material uncovered by the friction enhancing material. More preferably, the pattern is uniformly distributed over the semi-permeable material, or more preferably over the entire barrier film, thus allowing good slip resistance over its surface while not impeding permeation of sterilizing gases therethrough. Preferably, the portion of the semi-permeable material uncovered by the friction enhancing material is greater than 25 percent. A portion of the barrier film can be formed of an impermeable material that is impermeable to sterilizing gases and contaminating microorganisms, and the friction enhancing material can be located on the impermeable material.

The interior space may be defined by folding the barrier film, especially when the barrier film comprises CSR wrap.

### BRIEF DESCRIPTION OF THE DRAWINGS

FIG. 1 is a perspective view of a sterilization package according to the present invention;
FIG. 2 is a detail view of friction enhancing material on a surface of the package of FIG. 1;
FIG. 3 is a top plan view of an alternative sterilization package according to the present invention, shown prior to folding; and
FIG. 4 is a top plan view of the package of FIG. 3, shown folded.

### DETAILED DESCRIPTION

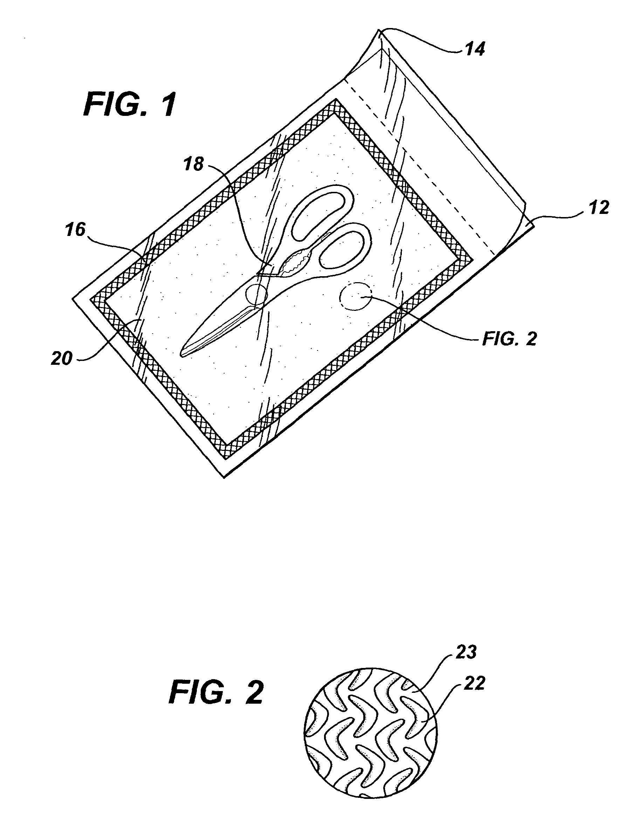

FIG. 1 shows a pouch 10 according to the present invention. The pouch comprises a first semi-permeable layer 12 formed of TYVEK™, spun-bonded polyethylene, or medical grade paper, and a second layer formed of laminated clear polyolefin and polyester 14. The semi-permeable film 12 and the clear film 14 are bonded together along a peripheral seal 16 formed with an adhesive. The seal can alternatively be formed with melt bonding or any other appropriate sealing methodology. An instrument 18 is received within an inner space 20 formed between the semi-permeable layer 12 and clear layer 14.

Turning also to FIG. 2, friction enhancing material 22 can be seen on an outer surface 23 of the clear layer 14. A similar pattern of friction enhancements 22 is placed upon the outer surface (not shown) of the semi-permeable film such as Tyvek™ or medical grade paper 12. The friction enhancing material 22 is placed in a pattern which does not interfere with the permeability of the semi-permeable layer 12 to sterilizing gases, nor does it overly impair the visibility of the instrument 18 through the clear layer 14. Although one particular pattern is shown, other patterns, either regular or irregular, can be employed.

The friction enhancing material 22 has a significantly higher static coefficient of friction than the underlying clear layer 14 and the semi-permeable layer 12. The preferred static coefficient is at least 0.2, more preferably 0.3 and over. Suitable materials include polymeric elastomers of thermolplastic or thermosetting types, plastics and potentially ceramics or metal. The material should be chosen based on its compatibility with traditional sterilization techniques as well as on its static coefficient of friction. The material 22 can be applied by printing, such as by silk screening, gravure or flexographic methods, so that the thickness of the deposition and printing pattern can be monitored and controlled. Other traditional coating methods such as spray coating or calendaring can be used as well, as long as the coating can be applied in a specified pattern. Before printing, the friction enhancing material 22 can be dissolved or suspended in water or solvent.

One particularly useful form of friction enhancing material 22 is silicone, such as polydimethyl siloxane or polydiphenylmethyl siloxane and derivatives which can be suspended in a solvent or aqueous solution and printed onto the semi-permeable film 12 and/or the clear layer 14. Silicone is particularly preferred as it resists the heat of steam, and most grades do not interfere with and are not adversely chemically affected by hydrogen peroxide or ethylene oxide. Silicone can also be selected with a fairly high static coefficient of friction.

The invention is not limited to any particular pouch construction. For instance, both layers can be made of a semi-permeable material, such as TYVEK™, medical grade paper or non-woven materials with bacterial filtration properties, such as CSR wraps. CSR wrap is typically formed of a non-woven polypropylene of single or laminated layers, such as SMS (spunbond-meltblown-spunbond) and is permeable to sterilizing vapors yet impermeable to potentially contaminating microorganisms. Alternatively, both layers can be made from a non-permeable material with one or more windows of semi-permeable material provided to allow penetration of sterilizing gases. The layers may be separate before the seal 16 is applied or may be one piece of material folded over upon itself and sealed together. Further, the friction enhancing material 22, while preferably applied to both sides of the pouch 10, may be applied merely to one side such as to the clear layer 14. If the pouch 10 is formed with windows of semi-permeable material, the friction enhancing material 22 can be applied to the remaining parts of the package without being applied to the windows.

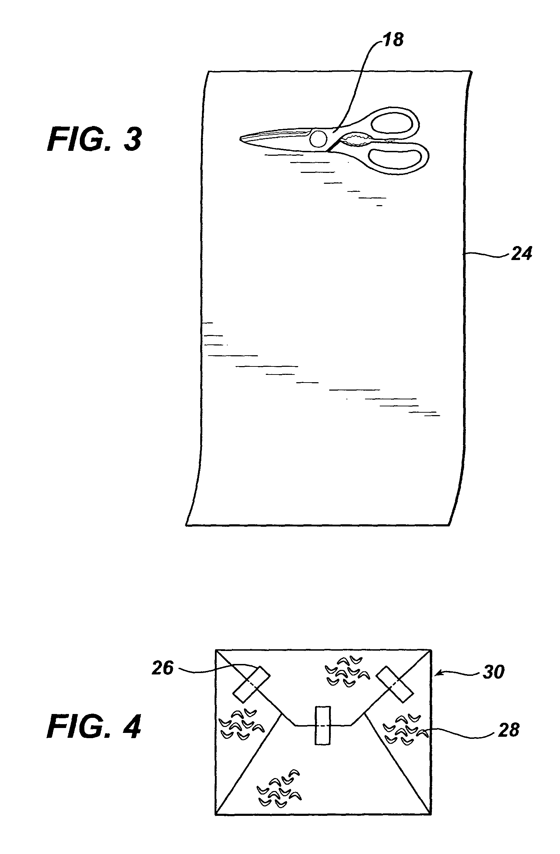

FIGS. 3 and 4 illustrate an alternative form of packaging. The instrument 18 is wrapped in a sheet or multiple sheets of CSR (central supply room) wrap 24. A folded configuration 30 as shown in FIG. 4 is held together by tape or sticker(s) 26. The tape or sticker 26 may be printed with a chemical indicator ink which changes color after exposure to the sterilizing gases. Friction enhancing material 28 is coated onto the CSR wrap 24 in a pattern to allow penetration of sterilizing gases through the CSR wrap 24 yet allow friction enhancement of the wrapped package 30.

CSR wrap is typically formed of a non-woven polypropylene of single or laminated layers, such as SMS spunbond-meltblown-spunbond) and is permeable to sterilizing vapors yet impermeable to potentially contaminating microorganisms. It is used to wrap sterilization trays and also to wrap individual instruments for sterilization during a sterilization procedure. The CSR wrap 24 of the present invention carries the friction enhancing material 28 so that when wrapped around the instrument 18 to form the package 30, the package 30 has slip resistance from the friction enhancing material 28. The friction enhancing material 28 need only be applied to one side of the CSR wrap 24, that side which will be on the outside of the finished package 30. Any pattern can be employed and need not be regular, but should be applied in such a fashion as to not impede the permeability of the CSR wrap 24 to the sterilizing gases, and to not interfere with the adhesion of the tape or sticker 26.

The invention now being fully described, it will be apparent to one of ordinary skill in the art that many modifications and changes can be made thereto without departing from the spirit or scope of the invention is defined in the following claims.

## Claims

1. A sterilization package for enclosing a device during a sterilization procedure and storing the device in sterile form thereafter, the package comprising:
a barrier film defining an interior space;
the barrier film sealing the interior space from contaminating microorganisms;
at least a portion of the barrier film being formed of a semi-permeable material which is permeable to sterilizing gasses and impermeable to contaminating microorganisms; and
at least a portion of an outer surface of the package having thereon a friction enhancing material.

2. A sterilization package according to claim 1 wherein the barrier film is flexible.

3. A sterilization package according to claim 2 wherein the barrier film is formed of two separate film panels sealed together by a peripheral seal.

4. A sterilization package according to claim 3 wherein both film panels have the friction enhancing material thereon.

5. A sterilization package according to claim 3 wherein at least one of the film panels consists of the semi-permeable material a portion of which has the friction enhancing material thereon.

6. A sterilization package according to claim 1 containing a medical instrument.

7. A sterilization package according to claim 1 wherein the friction enhancing material comprises an elastomer.

8. A sterilization package according to claim 7 wherein the elastomer comprises silicone.

9. A sterilization package according to claim 1 wherein a portion of the semi-permeable material has the friction enhancing material thereon.

10. A sterilization package according to claim 1 wherein the friction enhancing material is on the semi-permeable material in a pattern which leaves a portion of the semi-permeable material uncovered by the friction enhancing material.

11. A sterilization package according to claim 10 wherein the pattern is uniformly distributed over the semi-permeable material.

12. A sterilization package according to claim 11 wherein the pattern is uniformly distributed over the barrier film.

13. A sterilization package according to claim 10 wherein the portion of the semi-permeable material uncovered by the friction enhancing material is greater than 25 percent.

14. A sterilization package according to claim 1 wherein the interior space is defined by folding the barrier film.

15. A sterilization package according to claim 14 wherein the barrier film comprises CSR wrap.

16. A sterilization package according to claim 1 wherein a portion of the barrier film is formed of an impermeable material which is impermeable to sterilizing gases and contaminating microorganisms, and the friction enhancing material is located on the impermeable material.
